# EUROPEAN PATENT APPLICATION

(11) **EP 4 575 510 A1**
(43) Date of publication of application: **25.06.2025**
(21) Application number: 22955775.6
(22) Date of filing: 19.08.2022
(51) Int. Cl.: G01N 35/10, G01N 1/00

(54) **DISPENSING DEVICE, AND GENETIC TEST DEVICE PROVIDED WITH SAME**

(71) Applicant: HITACHI HIGH-TECH CORPORATION, Tokyo 105-6409 (JP)
(72) Inventor: SATO, Koma, Tokyo 100-8280 (JP); SATOH, Wataru, Tokyo 100-8280 (JP); ISOSHIMA, Nobuyuki, Tokyo 105-6409 (JP); SHIBAHARA, Masashi, Tokyo 105-6409 (JP); MAKINO, Yoko, Tokyo 105-6409 (JP); KUMAZAKI, Nobutaka, Tokyo 105-6409 (JP)
(74) Representative: Strehl & Partner mbB
(86) International application number: PCT/JP2022/031443
(87) International publication number: WO 2024/038603

(57) **Abstract**

Provided is, to reduce, when cartridges for reaction formed as a plurality of lanes are integrated, a risk of occurrence of cross contamination without providing, on the cartridges, a partition wall for partitioning the lanes and an air suction opening, a dispensing device including a plurality of dispensing machines, a dispensing device body that has the plurality of dispensing machines mounted thereto and is capable of moving along a direction of a plurality of lanes of a solution plate including a plurality of containers each of which accommodates a reagent or a sample and that are integrally formed along the lanes, a top plate for test on which the dispensing device body and the solution plate are placed, and a device cover that covers the dispensing device body in combination with the top plate for test, and a plurality of ribs that separate a space above the plurality of lanes of the container plate into spaces each for each of the plurality of lanes and cover the spaces are formed in the dispensing device body.

## Description

### Technical Field

The present invention relates to a multi-dispensing device which simultaneously handles a plurality of samples and a genetic testing device provided therewith and relates to a dispensing device which reduces a risk of cross contamination and a genetic testing device provided therewith.

### Background Art

In Patent Document 1, there is described a technology of including a multi-dispensing device which simultaneously handles a plurality of samples and using an independent reagent cartridge having a partition wall to manage each sample, thereby suppressing entry, to a solution containing another sample, of fine particles containing the sample and hence possibly causing cross contamination.

### Prior Art Document

### Patent Document

Patent Document 1: Japanese Patent No. 3630493

### Summary of the Invention

### Problem to be Solved by the Invention

The dispensing device is a device which suctions and discharges such liquid as a sample or a reagent in a determined amount in research, a test, or the like in the life science field. In particular, as a dispensing device mounted to a genetic testing device, a multi-dispensing device is used to be able to simultaneously test different samples. The multi-dispensing device is formed by disposing a plurality of independent dispensing machines in parallel.

When such a multi-dispensing device is used, different samples are simultaneously handled, and hence, it is required to suppress occurrence of what is generally called cross contamination, which is mixture of a certain sample with another sample. In a case where the cross contamination occurs, reliability of a test result decreases. In particular, in a genetic testing device that uses polymerase chain reaction (hereinafter referred to as PCR), a DNA, an RNA, or the like, which is a detection target, is exponentially amplified and detected, and hence, the cross contamination particularly poses a problem.

In Patent Document 1, there is described a structure which provides, when a plurality of rows of reaction lines (hereinafter referred to as lanes) for executing series of reaction processing for one sample are disposed side by side, partition walls for partitioning a space from an adjacent lane as well as air suction openings between the lanes for downward suctioning.

The structure described in Patent Document 1 is a structure which suppress, through the partition walls and the suction from the air suction openings, scattering of the fine particles, which contain the sample and possibly cause the cross contamination, toward an unintended direction and can easily be implemented through a cartridge structure which is independent for each lane. That is, it is only required to provide the partition wall at any one of left and right ends of a cartridge upper surface. By disposing such cartridges side by side in a lateral direction, a gap into which the air can flows is easily formed between the cartridges.

However, when such independent cartridges are used, there occurs such work that the cartridges are individually disposed one at a time at the time of the test and are removed when the test is finished. When this series of work is manually executed by a worker, touch on each portion of the device while the sample is attached to the fingers also possibly causes the occurrence of the cross contamination. Further, in a case where the sample has pathogenicity, it is desired that a frequency of access of the worker to the solution containing the sample or the like be as low as possible. For these reasons, it is desired to automate a process required for the test.

However, in order to automate the work of disposing, side by side, a plurality cartridges described in Patent Document **1,** it is required to repeat a procedure of extracting the cartridge from a storage place for the cartridges, moving the cartridge to a target position of each lane, then disposing the cartridge at the target position, and returning the cartridge to the storage place for as many number of times as the number of lanes, and hence, there is a risk in that a device configuration and control may become complicated.

Moreover, a common cartridge shape is unavoidably to be used, flow passage shapes for the lanes are the same, and hence, it is impossible to provide such a configuration as finely controlling flow passage cross-sectional areas.

Meanwhile, the device configuration and control can be simplified by integrating the individual cartridges for each lane, but an aluminum seal for sealing a reagent or the like is applied to a surface of the cartridge, and hence, it is difficult to form the partition walls and the air suction openings described in Patent Document **1.** It is thus difficult to suppress the movement of the fine particles that contain the sample between the lanes and that possibly cause the cross contamination, and hence, there poses a problem of an increase in risk.

The present invention provides a dispensing device and a genetic testing device provided therewith which solve the problem of the prior art described above, the dispensing device reducing, when cartridges for reaction formed as a plurality of lanes are integrated, a risk of occurrence of cross contamination without providing, on the cartridges, partition walls for partitioning the lanes and air suction openings.

### Means for Solving the Problem

In order to solve the problem of the prior art described above, in the present invention, a dispensing device includes a plurality of dispensing machines, a dispensing device body that has the plurality of dispensing machines mounted thereto and is capable of moving along a direction of a plurality of lanes of a solution plate including a plurality of containers each of which accommodates a reagent or a sample and that are integrally formed along the lanes, a top plate for test on which the dispensing device body and the solution plate are placed, and a device cover that covers the dispensing device body in combination with the top plate for test, in which a plurality of ribs that separate a space above the plurality of lanes of the container plate into spaces each for each lane of the plurality of lanes and cover the spaces are formed in the dispensing device body.

Moreover, in order to solve the problem of the prior art described above, in the present invention, a genetic testing device includes a DNA detection unit, a DNA amplification unit that amplifies a detection target detected by the DNA detection unit, an amplicon detection unit that quantitatively evaluates an amplification amount of the detection target amplified by the DNA amplification unit, a control unit that controls the DNA detection unit, the DNA amplification unit, and the amplicon detection unit, and an output unit that outputs a result of the quantitative evaluation of the amplification amount of the detection target performed by the amplicon detection unit, the DNA detection unit includes a dispensing device that dispenses a solution containing the detection target and a reagent to a container, the dispensing device includes a plurality of dispensing machines, a dispensing device body that has the plurality of dispensing machines mounted thereto and is capable of moving along a direction of a plurality of lanes of a solution plate including a plurality of containers each of which accommodates a reagent or a sample and that are integrally formed along the plurality of lanes, a top plate for test on which the dispensing device body and the solution plate are placed, and a device cover that covers the dispensing device body in combination with the top plate for test, and a plurality of ribs that separate a space above the plurality of lanes of the container plate into spaces each for each lane of the plurality of lanes and cover the spaces are formed in the dispensing device body.

### Advantages of the Invention

According to the present invention, in the dispensing device and the genetic testing device provided therewith, there is used the plate formed by integrating reagent/solution cartridges corresponding to a plurality of samples, and hence, the risk of cross contamination can be reduced without providing partition walls, air suction openings, and the like on a solution cartridge surface.

### Brief Description of the Drawings

FIG. 1 is a cross-sectional view of a dispensing device according to a first embodiment for illustrating a schematic configuration of the dispensing device.
FIG. 2 is a perspective view of a front surface of the dispensing device according to the first embodiment as viewed in a direction A-A of FIG. **1****.**
FIG. 3 is a cross-sectional view made in a direction indicated by arrows B-B of FIG. **1****.**
FIG. 4 is a perspective view of the dispensing device for illustrating a relation between a dispensing device body and dispensing machines, a solution plate, and rib structures according to the first embodiment.
FIG. 5 is a cross-sectional view that is made on a plane indicated by arrows C-C of FIG. 1 and that illustrates a relation between the solution plate and the rib structures of the dispensing device according to a second embodiment.
FIG. 6 is a cross-sectional view that is made on the plane indicated by the arrows C-C in FIG. 1 and that illustrates a relation between the solution plate and the rib structures of the dispensing device according to the second embodiment, which is different from that of FIG. 5.
FIG. 7 is a perspective view for illustrating a relation between the dispensing machines and the rib structures of the dispensing device according to a third embodiment.
FIG. 8 is a cross-sectional view made on the plane indicated by the arrows C-C in FIG. 1 and illustrating the relation between the solution plate and the rib structures of the dispensing device according to a fourth embodiment.
FIG. 9 is a block diagram for illustrating a configuration of a genetic testing device according to a fifth embodiment provided with any one of the dispensing devices described in the first embodiment to the fourth embodiment.

### Modes for Carrying Out the Invention

There is a genetic testing device having such a configuration that test lanes are disposed in parallel to simultaneously test a plurality of samples. In this configuration provided with the test lanes, dispensing machines which simultaneously inject samples or reagents into a solution plate corresponding to a plurality of test lanes accompany discharging/stirring by the dispensing machines and movements of the dispensing machines. At this time, there possibly occurs cross contamination in which fine particles are scattered by an airflow and are mixed with other samples.

Meanwhile, the present invention has such a configuration that wall-surface rib structures are provided in a portion positioned below dispensing machines and opposing a solution plate to form a guide for an airflow to separate flows from each other, thereby suppressing the cross contamination of the fine particles in the dispensing machines.

The present invention provides a dispensing device which integrates reagent/solution cartridges corresponding to a plurality of samples and can reduce a risk of cross contamination without providing, on a solution cartridge surface, partition walls, air suction openings, and the like and a genetic testing device provided therewith.

A description is now given of embodiments of the dispensing device and the genetic testing device according to the present invention with reference to the drawings. Note that components having identical functional configurations in a description given below and the drawings are denoted by identical reference symbols to omit a redundant description.

Note that the present invention is not to be interpreted as being limited to described contents of the embodiments given below. It is easily understood by those skilled in the art that a specific configuration of the present invention can be modified within a scope not departing from the idea or the purport of the present invention.

### First Embodiment

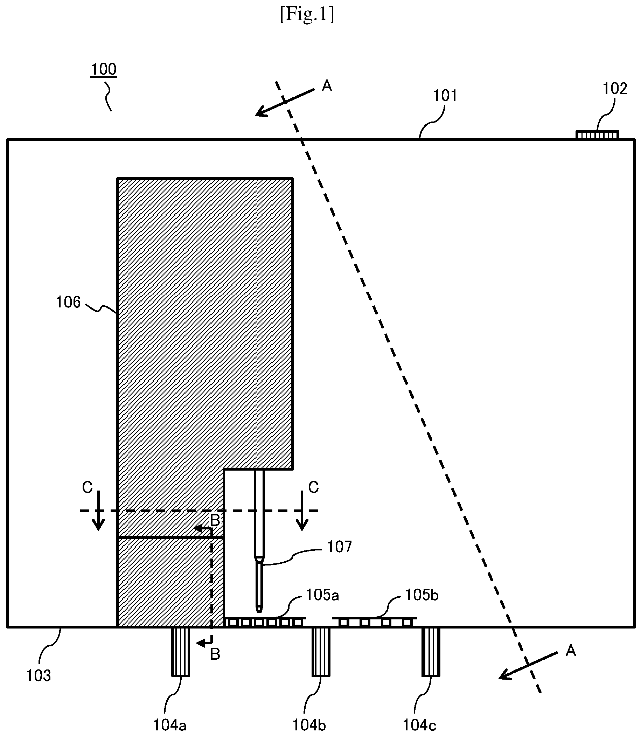

FIG. 1 illustrates a schematic configuration of a dispensing device 100 according to the first embodiment used for a genetic testing device and the like. The dispensing device 100 is formed by a dispensing device body 106, to which a plurality of dispensing machines 107 are mounted, being covered with a top plate 103 for test and a device cover 101.

On the device cover 101, an intake opening 102 which causes the air to flow inward from the outside of the device is provided. On the top plate 103 for test, there are disposed, in addition to exhaust openings 104a to 104c for exhausting, by unillustrated exhaust mean, the air inside the device including the dispensing device body 106 covered with the device cover 101, a solution plate 105a for accommodating reagents and the like for executing test processing and a solution plate 105b for accommodating samples.

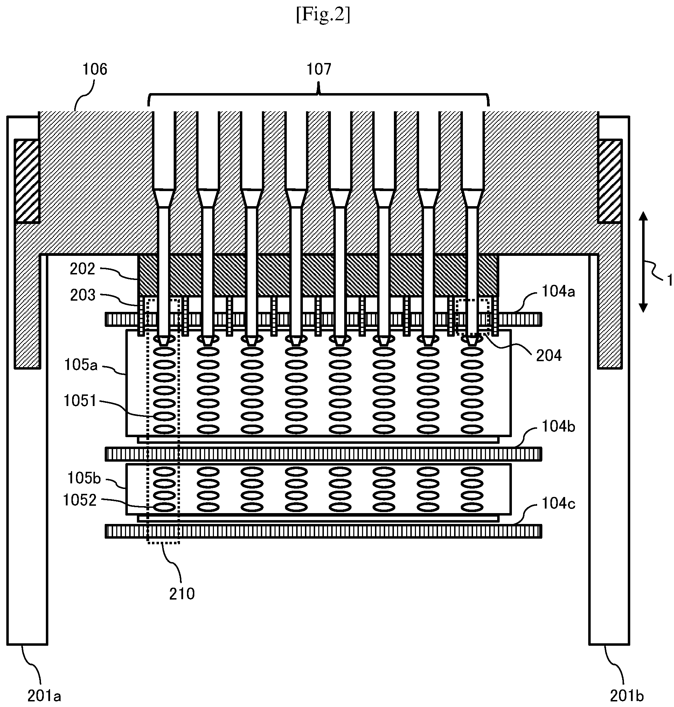

The solution plate 105a functions similarly to conventional solution cartridges described in Patent Document 1, but does not include individual cartridges for each reaction lane 210, and a large number of containers 1051 for accommodating the solution for executing the test processing are formed in each of a length direction and a width direction and are integrated, as illustrated in FIG. 2. Moreover, in the solution plate 105b, a large number of containers 1052 for accommodating the samples are formed and are integrated. It is assumed that the disposition of the solution plate 105a for executing the test processing and the solution plate 105b for accommodating the samples can be changed as desired according to a sequence of the test processing and the device configuration.

The exhaust openings 104a to 104c can each have a duct structure formed of, for example, a member in a plate shape made of metal, resin, or the like, and may also be a flow passage, such as a gap between members, which appears as a result of construction of the device and through which an airflow passes.

The dispensing device body 106 has the plurality of the dispensing machines 107 mounted thereto as described in FIG. 2 and is configured to be movable in a left-right direction of the page of FIG. **1****.** The dispensing machines 107 move as the dispensing device body 106 moves in the left-right direction. Moreover, the dispensing machine 107 is configured to be movable in the up-down direction of the page of FIG. 1 and has a function of dispensing liquid. That is, the dispensing machine 107 has a function of, in cooperation with movement control for the dispensing device body 106 described before, suctioning a certain quantity of the sample or the reagent, for example, accommodated in the container of any one of the solution plates 105a and 105b and discharging the sample or the reagent, for example, in another container. Moreover, the dispensing machine 107 may have a discharging/stirring function of repeating the suction and the discharge of the liquid accommodated in the container of any one of the solution plates 105a and 105b and thereby mixing the liquid.

A schematic configuration of the first embodiment is illustrated in FIG. 2 as viewed from a cross section indicated by A-A of FIG. **1****.** The dispensing device body 106 is configured to be movable in a direction indicated by an arrow 11 in FIG. 2 while guided by linear guides 201a and 201b installed on the device top plate.

A rectangle 210 in a dotted line indicates the reaction lane 210 in the present embodiment, and one sample is processing within one reaction lane 210. It is assumed that the present embodiment has such a configuration as the solution plate 105a in which the plurality of containers 1051 which accommodate the reagents and the solutions required for the processing for the reaction lane 210 are integrally formed, which is different from the solution cartridges described in Patent Document **1.** Similarly, it is assumed that the solution plate 105b which holds sample solutions also has such a configuration that the plurality of containers 1052 are integrally formed. As described above, with the configuration that the solution plates 105a and 105b are formed by the plurality of containers 1051 and 1052 being integrated, respectively, automatic disposition to a predetermined position is facilitated, and time required to dispose each of the plurality of containers 1051 and 1052 can be reduced, which contributes to automation of genetic test processing work.

In a lower portion of the dispensing device body 106 illustrated in FIG. **2****,** there is installed a structure 202 including magnets and the like required for a sample operation using magnetic particles in the reaction processing. Further, a plurality of rib structures 203 which extend in a vertical direction with respect to a bottom surface of the structure 202 are formed on a side of the structure 202 and are disposed in such a manner as to partition a space for each reaction lane 210. For example, when the structure 202 including the magnets and the like is present above the solution plate 105a, the rib structures 203 are disposed in such a manner as to partition, for each reaction lane 201, a space between an upper surface of the solution plate 105a and the bottom surface of the structure 202. The same applies to the solution plate 105b.

The solution plates 105a and 105b, as well as other components required for the genetic testing device, and the rib structures 203 are required to be disposed in such a manner as to avoid interference at the time of operation. For example, it is required to maintain a certain distance between the solution plates 105a and 105b and lower ends of the rib structures 203 to avoid contact. However, as described later, in order to form the space below the structure 202 as spaces independent of each other as much as possible for each reaction lane 210, it is desired that the distance between the lower ends of the rib structures 203 and the solution plate 105a be short.

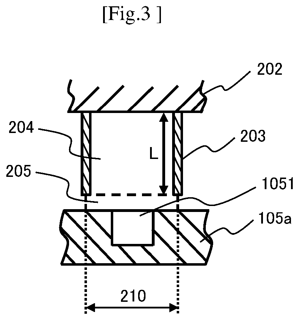

Meanwhile, as illustrated in FIG. 3 as a cross-sectional view made on a plane indicated by arrows B-B of FIG. 1, a length L of the rib structures 203 is set such that a cross-sectional area of a space 204 surrounded by the bottom surface of the structure 202 including the magnets and the like and the rib structures 203 adjacent to each other is sufficiently wider than a cross-sectional area of a space 205 formed of a surface formed by the lower ends of the rib structures 203 adjacent to each other being connected to each other and the surface of the solution plate 105a. With this configuration, the airflow passing below the structure 202 substantially passes through the space 204 for each reaction lane 210, and hence, it is possible to separate the airflow for each reaction lane.

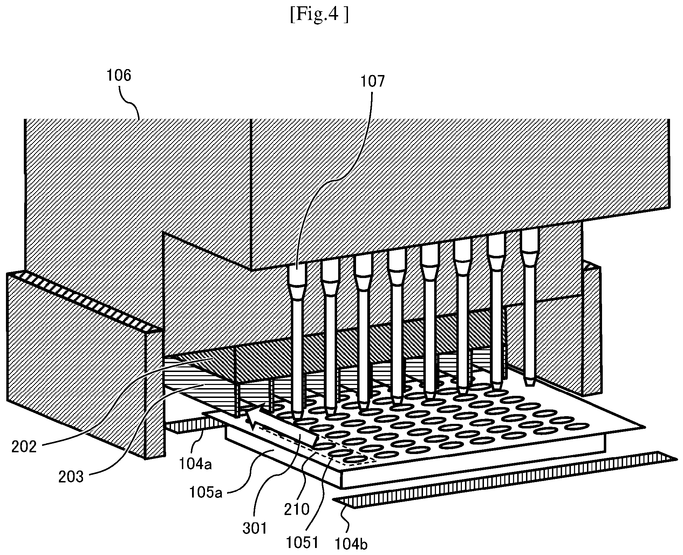

FIG. 4 is a perspective view of a periphery of the dispensing device body 106 in FIG. **2****.** The airflow over the solution plate 105a is straightened by the rib structures 203 and hence flows substantially along the reaction lane 210 as an arrow 301 indicates. This airflow is a part of a flow structure which flows into the device from the intake opening 102, illustrated in FIG. 1, for causing the air to flow from the outside of the device to the inside and flows out from the exhaust openings 104a, 104b, and 104c. Thus, fine particles possibly generated by scattering of the solution that is attached to a distal end of the dispensing machine 107 and that contains the sample and fine particles possibly generated at the time of processing executed on the solution plate 105a, that is, fine particles possibly constituting a cross-contamination source, can be guided by the airflow flowing toward a direction of the arrow 301 and can be collected from the exhaust opening 104a.

Thus, according to the present embodiment, it is possible to reduce a risk of the occurrence of the cross contamination between the reaction lanes 210 adjacent to each other.

Moreover, according to the present embodiment, the rib structures 203 are installed on the bottom surface of the structure 202 including the magnets and the like, and the upper surfaces of the solution plates 105a and 105b can thus be flat, so that the plurality of solution plates 105a or 105b can be stored in a stacked state. As a result, a storage space for the plurality of solution plates 105a or 105b can be smaller than that in a case in which the upper surface is not flat.

In the present embodiment, when the rib structures 203 are installed on the bottom surface of the structure 202 including the magnets and the like, the structure 202 may be made of metal, and the rib structures 203 may be cut, thereby integrally forming the rib structures 203 with the structure 202. Moreover, the rib structures 203 may be formed independently of the structure 202, and each rib structure 203 may be formed of a component in a plate form bent into an L shape. In this case, the components bent into the L shape may be adhered to or fixed through screws or the like to the structure 202 to form the rib structures 203. As another example, by providing a slide mechanism between the structure 202 and a component forming the rib structure 203, the rib structure 203 can be configured to be detached from the structure 202 by sliding the rib structure 203.

Moreover, in FIG. 2 and FIG. **4****,** the shape of the rib structure 203 is a rectangular parallelepiped, and hence, the cross-sectional shape of the space formed by the rib structures 203 adjacent to each other is substantially rectangular. However, the cross-sectional shape of the rib structure 203 may also be changed as desired according to necessity. For example, there can be made such a change that a joined portion between the structure 202 and the rib structure 203 has a fillet shape. Moreover, the cross-sectional shape may be changed for each reaction lane 210, thereby being able to change a cross-sectional area distribution. As a result, it is possible to control, for each reaction lane, a pressure loss of the space formed of the structure 202 and the rib structures 203, thereby being able to more flexibly control the airflow. Moreover, other than the space of the open type formed of the rib structures, there can be made such a change that a tubular cross-sectional shape is formed to more clearly divide the flow passage for each lane and the like.

### Second Embodiment

A description is now given of a configuration according to a second embodiment of the present invention. The present embodiment focuses on the rib structures 203 according to the first embodiment and achieves balance among flow passage resistances of the spaces formed of the rib structures 203 for the reaction lanes 210, thereby effectively uniformizing the flowrates.

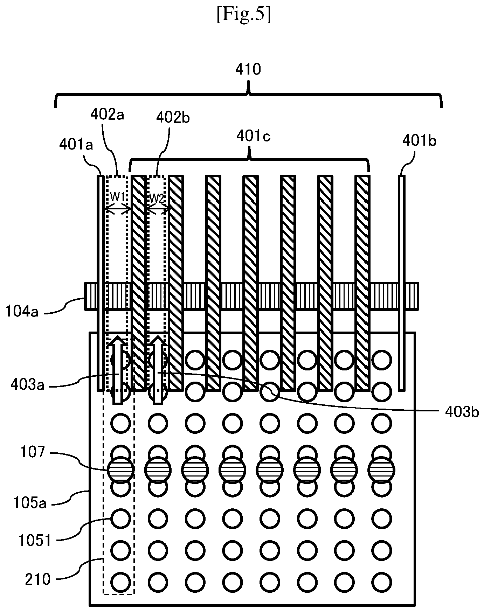

FIG. 5 illustrates a configuration in the second embodiment corresponding to the rib structures 203 on the cross section made on a plane indicated by B-B of FIG. 1 and a configuration of a periphery of the exhaust opening 104a. In the present embodiment, rib structures 401a and 401b which are among the rib structures 410 and correspond to the reaction lanes 210 at left and right ends among the plurality of reaction lanes 210 formed on the solution plate 105a are thick rib structures, and the rib structures 401c which partition the spaces for the reaction lanes 210 on the inner side of the reaction lanes 210 at the left and right ends (inner reaction lanes 210 between the reaction lanes 210 at the left and right ends) are relatively thin rib structures.

With this configuration, a width W1 of the flow passage 402a for each of the reaction lanes 210 at the left and right ends among the plurality of reaction lanes 210 is wider than a width W2 of the reaction lanes 210 on the inner side of the reaction lanes 210 at the left and right ends, and hence, a flow passage cross sectional area of the flow passage 402a of each of the reaction lanes 210 at the left and right ends is wider than a flow passage cross sectional area of the flow passage 402b of each of the reaction lanes 210 on the inner side of the reaction lanes 210 at the left and right ends. As a result, it is possible to achieve such a configuration that a pressure loss of the flow passage 402a of each of the reaction lanes 210 at the left and right ends is smaller than a pressure loss of the flow passage 402b of each of the reaction lanes 210 on the inner side of the reaction lanes 210 at the left and right ends.

When the exhaust opening 104a is a rectangle, the pressure loss is large at left and right ends in a length direction (left-right direction in FIG. 5), and hence, the air less likely to flow. But, by employing the rib structures 410 described in the present embodiment, it is possible to balance the pressure losses in the flow passages in which air flows from the reaction lanes 210 and passes through the exhaust opening 104a. As a result, it is possible to uniformize the flowrates of the airflow 403a passing through the reaction lane 210 at the left end and the airflow 403b passing through the adjacent reaction lane 210, and hence, it is possible to carry, on the stabilized airflows, the fine particles that contain the sample and the like and that constitute the cross-contamination source, thereby more efficiently collecting the fine particles from the exhaust opening 104a.

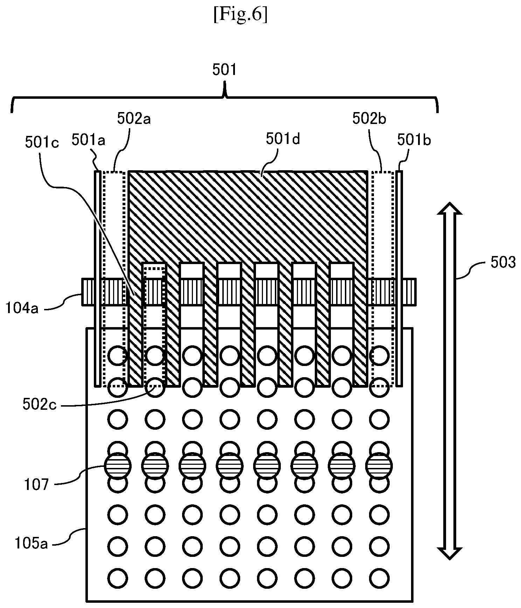

Moreover, the cross-sectional shapes of the reaction lanes 210 may be changed to have a distribution of the cross-sectional area, thereby being able to adjust the pressure losses of the flow passages. An example is illustrated in FIG. 6. In FIG. **6****,** rib structures 501c corresponding to the rib structures 401a and 401b corresponding to the reaction lanes 210 at the left and right ends illustrated in FIG. 5 are connected to each other through a block 501d on a housing inner side to fill the space.

Meanwhile, it is assumed that the rib structures 501a and 501b corresponding to the left and right ends of the reaction lanes 210 do not fill the space on the housing inner side. The dispensing device body 106 including the rib structures 501 illustrated in FIG. 6 moves toward directions of an arrow 503. Thus, with the rib structure 501 as illustrated in FIG.6, the position of the dispensing machine effective as the flow passage can be controlled for each reaction lane 210.

That is, the flow passage cross-sectional areas of the flow passages 502a and 502b of the reaction lanes 210 at the left and right ends are constant, but the flow passages 502c of the inner reaction lanes 210 between the reaction lanes 210 at the left and right ends are restricted according to the position of the dispensing device body 106, so that the flow passage cross-sectional areas decrease, and the flow passage resistances increase. As a result, it is possible to control the pressure loss in the flow passage for each reaction lane 210, thereby being able to flexibly control the airflow.

According to the present embodiment, in addition to the effects described in the first embodiment, it is possible to increase a degree of freedom in a flow passage design for each reaction lane 210 by providing the straightening structure to each of the rib structures 203 formed on the bottom surface of the structure 202 including the magnet and the like. In the conventional structure of disposing the solution cartridges side by side to form the reaction lanes, it has been difficult to flexibly execute the flow passage design as described above.

Note that, in the example illustrated in FIG. 5, the example in which the thickness of the rib structures 401a and 401b are thinner than the thickness of the rib structures 402c on the inner side is described, but the configuration is not limited to this example, and, as long as the width W1 of the flow passages 402a of the reaction lanes 210 at the left and right ends can be set to be wider than the width W2 of the reaction lanes 210 on the inner side of the reaction lanes 210 at the left and right ends, the thickness of the rib structures 401a and 401b may be the same as or thicker than that of the rib structures 402c on the inner side of the reaction lanes 210 at the left and right ends. The same applies to the configuration described in FIG. 6.

### Third Embodiment

A description is now given of a configuration according to a third embodiment of the present invention. The present embodiment focuses on the structure 202 including the magnets and the rib structures 203 in the first embodiment, and the airflows passing through the space formed by the structure 202 and the rib structures 203 are more effectively straightened.

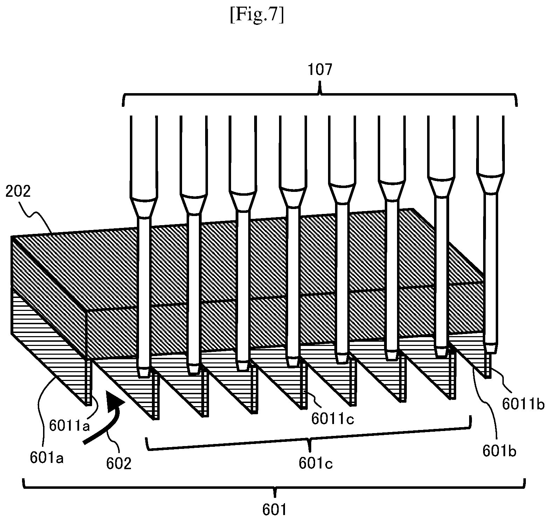

FIG. 7 illustrates the configuration of the structure 202 including magnets and rib structures 601 in the third embodiment. This configuration is the same as that of the first embodiment in such a point that the space below the bottom surface of the structure 202 including the magnets is partitioned for each reaction lane 210 (see FIG. 2) by disposing the rib structures 601 on the bottom surface of the structure 202.

However, for rib structures 601a and 601b at the left and right ends disposed on the outer side of the reaction lanes 210 at the left and right ends, a position the same as a front surface of the structure 202 is set to distal end portions 6011a and 6011b of the rib structures 601a and 601b, respectively. Meanwhile, for inner-side rib structures 601c between the rib structures 601a and 601b at the left and right ends, distal end portions 6011c are set to a position closer to the dispensing machines 107.

That is, the distal end portions 6011c of the inner-side rib structures 601c protrude toward the side of the dispensing machines 107 more than the distal end portions 6011a and 6011b of the rib structures 601a and 601b at the left and right ends.

It is considered that, in a vicinity of a distal end of the dispensing machine 107 close to the side of the solution plates 105a and 105b (see FIG. 2), the fine particles constituting the cross-contamination source are generated by influence of an airflow and the like during the test processing and the vicinity hence highly probably constitutes a scattering source. Thus, it is possible to exert the straightening effect for each reaction lane 210 from an upstream side of the airflow by extending the distal end portion 6011c to the vicinity of the dispensing machine 107 as the rib structure 601c.

Moreover, it is possible to form flow passages each of which causes an airflow in a lateral direction to flow toward the reaction lanes 210 at the left and right ends as indicated by a direction of an arrow 402, by setting the distal end portions 6011a and 6011b to the position the same as the front surface of the structure 202 as in the rib structures 601a and 601b. As described above, it is possible to set as desired the positions of the distal end portions 6011a, 6011b, and 6011c of the rib structures 601 appropriate for each reaction lane 210.

Moreover, it is possible to use such a mechanism as an actuator to dynamically change the positions of the distal end portions 6011a, 6011b, and 6011c of the rib structures 601a to 601c. By enabling the dynamic change in positions of the distal end portions 6011a, 6011b, and 6011c, the fine particles constituting the cross-contamination source can more flexibly be collected, and constraint on object interference is alleviated as well when installation and replacement of the solution plates 105a and 105b, the dispensing machines 107, and the like are automatically executed.

According to the present embodiment, in addition to the effects described in the first embodiment and the second embodiment, the straightening effect for each reaction lane 210 can be exerted from the upstream side of the airflow on which the fine particles constituting the cross-contamination source are generated due to the influence of the airflow and the like during the test processing and which hence highly probably constitutes the scattering source.

### Fourth Embodiment

A description is now given of a configuration according to a fourth embodiment of the present invention. The present embodiment focuses on the exhaust opening 104a in a device rear portion in the first embodiment and more effectively straightens the airflow passing through the space formed by the structure 202 and the rib structures 203 described in FIG. **2****.**

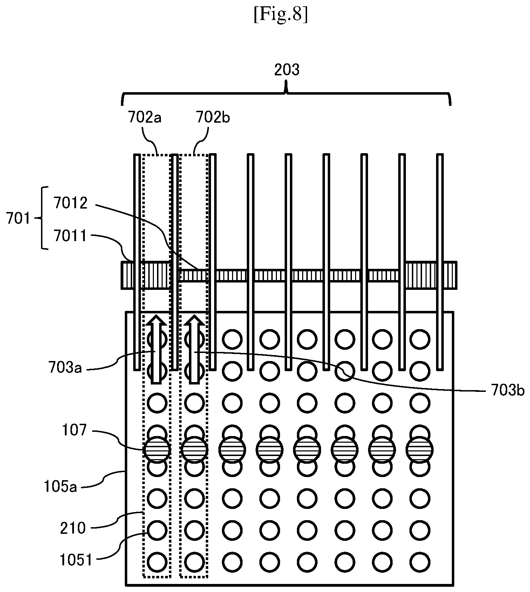

FIG. 8 illustrates a configuration of the rib structures 203 and a periphery of an exhaust opening 701 in the fourth embodiment and corresponds to a configuration of the rib structures 203 and the periphery of the exhaust opening 104a as viewed from the cross section indicated by the arrows C-C illustrated in FIG. 1 in the first embodiment. The configurations of the rib structures 203, the solution plate 105a, and the dispensing machines 107 in FIG. 8 are the same as those described in the first embodiment.

It is assumed that the exhaust opening 701 in the present embodiment has a flow passage shape having an opening shape changing in a device left-right direction (left-right direction of the page) as opening regions 7011 and 7012. In FIG. **8****,** the airflow which passes through a reaction lane 702a at the left end substantially passes through the opening region 7011 having a wide flow passage area of the exhaust opening 701, and the airflow which passes through an adjacent reaction lane 702b substantially passes through the opening region 7012 having a narrow flow passage area of the exhaust opening 701. In general, the pressure loss is relatively large at both ends in the length direction of a rectangular pipeline, while the pressure loss is small in a vicinity of the center. Thus, achieving such a flow passage shape that the flow passage area is wide at both ends and the flow passage area is narrow at the center as 701, the pressure loss can be balanced. Therefore, it is possible to uniformize the flowrates of the airflow 703a passing through the lane at the left end and the airflow 703b passing through the adjacent lane, and hence, it is possible to carry, on the stabilized airflows, the fine particles that contain the sample and the like and that constitute the cross-contamination source, thereby more efficiently collecting the fine particles.

In FIG. 8, there is illustrated an example in which a flow passage cross sectional area of the exhaust opening 701 changes stepwise in the device left-right direction, but the flow passage cross sectional area may have any shape, and may have a flow passage cross sectional area distribution which changes continuously. Moreover, the exhaust opening 701 may be configured as a hole provided to the top plate 103 for test (see FIG. 1) as well as a duct formed of resin, metal, or the like.

Further, the exhaust opening 701 may be configured such that an entire region thereof is formed at a width of the opening region 7011 and a member which increases an air resistance, for example, a mesh plate, may be installed in a portion corresponding to the opening region 7012.

With the configuration described above, according to the present embodiment, effects similar to those described in the first embodiment to the third embodiment can be obtained.

### Fifth Embodiment

A description is now given of a configuration according to a fifth embodiment of the present invention. The present embodiment is an example in which the dispensing device 100 described in any one of the first embodiment to the fourth embodiment is applied to a genetic testing device (hereinafter referred to as a PCR device) 800 which uses the PCR reaction. Note that the dispensing devices 100 described herein include the dispensing devices obtained by making the changes as described in the second embodiment to the fourth embodiment to the dispensing device 100 described in the first embodiment.

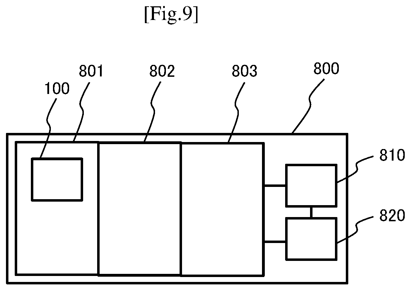

FIG. 9 is a schematic configuration of the PCR device 800 which uses the dispensing device 100 in the fifth embodiment. The PCR device 800 includes a DNA extraction unit 801 provided with the dispensing device 100, a DNA amplification unit 802, an amplicon detection unit 803, a control unit 810 which controls the entire device, and an output unit 820 which outputs a result of the test.

The DNA extraction unit 801 executes mixing of a solution or the like containing a detection target such as a DNA or an RNA and a reagent, magnetic particles, and the like with each other, discharging/stirring, dispensing, and the like to extract the detection target. In the DNA extraction unit 801, the number of operations such as the discharging/stirring and the dispensing which use the dispensing device 100 and the movements and the like of the dispensing machines is large, a risk of the contamination is relatively high, and hence, the straightening structure of the reaction lanes 210 achieved by the dispensing devices 100 described in the first embodiment to the fourth embodiment is particularly effective.

In the DNA amplification unit 802, the solution containing the detection target such as a DNA or an RNA extracted by the DNA extraction unit 801 is dispensed and is placed in a device called a thermal cycler (not illustrated). The thermal cycler is a device which can execute high-speed and precise temperature adjustment, executes temperature adjustment according to the detection target or the reagent which is used, to execute such operations as denaturation, annealing, and extension, thereby amplifying a DNA or an RNA.

Moreover, in the amplicon detection unit 803, a container containing the detection target such as a DNA or an RNA which is amplified by the DNA amplification unit 802 is dispensed, and fluorescence intensity and the like are measured, thereby quantitatively evaluating an amplification amount of the detection target.

As a result of the use of the dispensing device 100 described in any one of the first embodiment to the fourth embodiment in the genetic testing device 800 which uses the PCR reaction as described in the fifth embodiment, the risk of the cross contamination can be reduced, thereby being able to increase reliability of a genetic test result.

The description has been given of the invention made by the present inventors, on the basis of the embodiments, but the present invention is not limited to the embodiments, and it should be understood that the present invention can be modified in various ways within a scope not departing from the gist thereof. For example, the above-mentioned embodiments are detailed for an easy-to-understand description of the present invention, and the present invention is not necessarily limited to a case including all of the described configurations. Moreover, to a part of the configuration of each of the embodiments, another configuration can be added, the part can be deleted, or the part can be replaced by another configuration.

### Description of Reference Symbols

100: Dispensing device
101: Device cover
102: Intake opening
103: Top plate for test
104a, 104b, 104c, 701: Exhaust opening
105a, 105b: Solution plate
106: Dispensing device body
107: Dispensing machine
201a, 201b: Guide
202: Structure
203, 410, 501, 601: Rib structure
210: Reaction lane
800: Genetic testing device

## Claims

1. A dispensing device comprising:
a plurality of dispensing machines;
a dispensing device body that has the plurality of dispensing machines mounted thereto and is capable of moving along a direction of a plurality of lanes of a solution plate including a plurality of containers each of which accommodates a reagent or a sample and that are integrally formed along the lanes;
a top plate for test on which the dispensing device body and the solution plate are placed; and
a device cover that covers the dispensing device body in combination with the top plate for test,
wherein a plurality of ribs that separate a space above the plurality of lanes of the solution plate into spaces each for each lane of the plurality of lanes and cover the spaces are formed in the dispensing device body.

2. The dispensing device according to claim **1,**
wherein an intake opening is formed in the device cover, an exhaust opening is formed in the top plate for test, and the plurality of ribs are formed such that a part of air that flows from the intake opening into an inside of the device cover and flows out from the exhaust opening flows along spaces formed by the plurality of ribs.

3. The dispensing device according to claim 2,
wherein the plurality of ribs are formed such that resistances against the air flowing in a vicinity of a surface of the solution plate along the spaces formed by the plurality of ribs are different from one another according to positions of the plurality of ribs.

4. The dispensing device according to claim 3,
wherein intervals between the plurality of ribs that separate the space above the plurality of lanes of the solution plate into the spaces each for each lane of the plurality of lanes and cover the spaces are different from one another according to the position of each rib of the plurality of ribs.

5. The dispensing device according to claim 4,
wherein the interval of the ribs that are among the plurality of ribs and cover the space above the lane in an end portion among the plurality of lanes of the solution plate is formed in such a manner as to be wider than the interval of the ribs that are among the plurality of ribs and cover the space above the lane on an inner side of the lane in the end portion.

6. The dispensing device according to claim **1,**
wherein the plurality of dispensing machines are mounted to the dispensing device body in correspondence to the plurality of lanes, and intervals of the plurality of ribs that separate the space above the plurality of lanes of the solution plate into the spaces each for each lane of the plurality of lanes and cover the spaces and the dispensing machine vary according to positions of the plurality of ribs.

7. The dispensing device according to claim **2,**
wherein the exhaust opening of the top plate for test is formed in such a manner as to cross the plurality of lanes of the solution plate, and a vicinity to an end portion of the exhaust opening in a direction crossing the plurality of lanes of the solution plate is formed in such a manner as to be lower in resistance against the air passing through the exhaust opening than an inner side of the vicinity to the end portion.

8. A genetic testing device comprising:
a DNA detection unit;
a DNA amplification unit that amplifies a detection target detected by the DNA detection unit;
an amplicon detection unit that quantitatively evaluates an amplification amount of the detection target amplified by the DNA amplification unit;
a control unit that controls the DNA detection unit, the DNA amplification unit, and the amplicon detection unit; and
an output unit that outputs a result of the quantitative evaluation of the amplification amount of the detection target performed by the amplicon detection unit,
wherein the DNA detection unit includes a dispensing device that dispenses a solution containing the detection target and a reagent to a container,
the dispensing device includes
a plurality of dispensing machines,
a dispensing device body that has the plurality of dispensing machines mounted thereto and is capable of moving along a direction of a plurality of lanes of a solution plate including a plurality of containers each of which accommodates a reagent or a sample and that are integrally formed along the plurality of lanes,
a top plate for test on which the dispensing device body and the solution plate are placed, and
a device cover that covers the dispensing device body in combination with the top plate for test, and
a plurality of ribs that separate a space above the plurality of lanes of the solution plate into spaces each for each lane of the plurality of lanes and cover the spaces are formed in the dispensing device body.
